# EUROPÄISCHE PATENTANMELDUNG

(11) **EP 0 629 638 A1**
(43) Veröffentlichungstag der Anmeldung: **21.12.1994**
(21) Anmeldenummer: 94108434.5
(22) Anmeldetag: 01.06.1994
(51) Int. Cl.: C08F 8/46, C08F 8/32, C10M 149/14

(54) **Verfahren zur Herstellung von Polyisobutylbernsteinsäureanhydriden**

(30) Priorität: 14.06.1993 DE 4319672
(71) Anmelder: BASF Aktiengesellschaft, D-67063 Ludwigshafen (DE)
(72) Erfinder: Rath, Hans Peter, Dr., D-67269 Gruenstadt (DE); Mach, Helmut, Dr., D-69115 Heidelberg (DE); Schwahn, Harald, Dr., D-69168 Wiesloch (DE); Schreyer, Peter, Dr., D-69469 Weinheim (DE); Fehr, Erich K., Dr., D-34246 Vellmar (DE)

(57) **Zusammenfassung**

Herstellung von Polyisobutylbernsteinsäureanhydriden aus Polyisobuten und Maleinsäureanhydrid, indem man Polyisobuten mit einem mittleren Molekulargewicht M_{N} von 600 - 5000 und einem Gehalt an endständigen Doppelbindungen von mindestens 50 % mit Maleinsäureanhydrid in einer Reinheit von mindestens 99 % im Verhältnis von 1,1 : 1 bis 6 : 1 mol Maleinsäureanhydrid zu Polyisobuten bei 140 - 200°C umsetzt.

## Beschreibung

Die vorliegende Erfindung betrifft ein verbessertes Verfahren zur Herstellung von Poiyisobutylbernsteinsäureanhydriden aus Polyisobuten und Maleinsäureanhydrid, dadurch gekennzeichnet, daß man Polyisobuten mit einem mittleren Molekulargewicht M_{N} von 600 - 5000 und einem Gehalt an endständigen Doppelbindungen von mindestens 50 % mit Maleinsäureanhydrid in einer Reinheit von mindestens 99 % im Verhältnis von 1,1 : 1 bis 6 : 1 mol Maleinsäureanhydrid zu Polyisobuten bei 140 - 200°C umsetzt. Weiterhin betrifft die Erfindung Polyisobutylbernsteinsäureamide und -imide, die aus dem nach dem beschriebenen Verfahren erhältlichen Polyisobutylbernsteinsäureanhydriden zugänglich sind. Ferner betrifft die Erfindung die Verwendung der Polyisobutylbernsteinsäureamide und -imide als Kraftstoff- und Schmieröladditive sowie Kraftstoffe und Schmieröle, die die genannten Verbindungen enthalten.

Es ist allgemein bekannt, daß durch Zusatz verschiedener Additive zum Kraftstoff die Reinhaltung von Vergasern, Einspritzdüsen, Ansaugrohren und Einlaßsystemen von Otto-Motoren bzw. Dieselmotoren verbessert werden kann (siehe z.B. M. Rosenbeck "Katalysatoren, Tenside, Mineralöladditive", J. Falbe, U. Hasserodt, S. 223 ff., Thieme Verlag, Stuttgart 1978 und Ullmann's Encyclopedia of Industrial Chemistry, Vol. A 16, Seite 719 ff., VCH Verlagsgesellschaft 1990). Die Anwesenheit von Additiven wie Polyisobutylbernsteinsäureimiden führt zur Erhöhung von im Brennraum unerwünschten Ablagerungen (siehe z.B. "Deposits in Gasoline Engines - A Literature Review", G.F. Kalghatgi, SAE-Paper 902105 (1990)). Solche Ablagerungen tragen wesentlich zum Anstieg des Octanzahlbedarfes bei. Eine Minimierung dieser Ablagerungen sollte deshalb neben einer verbesserten Reinhaltung von Vergaser-, Einspritz- und Einlaßsystemen das Ziel einer Neuentwicklung von Additiven sein.

Polyisobutylbernsteinsäureimide finden weiterhin als sogenannte aschefreie Dispergatoren breite Anwendung im Schmierstoffbereich. In Motorölen für Otto- und Dieselmotoren sind sie in Mengen bis zu 10 Gew.-% enthalten und sollen hier die Agglomeration, Ausfällung und Ablagerung von unlöslichen Teilchen verhindern, die im allgemeinen als Schlammbildung und -ablagerung bezeichnet wird.

Die Dispergierwirkung dieser Zusätze ist für diese Anwendung die wichtigste Eigenschaft.

Polyisobutylbernsteinsäureimide werden im allgemeinen aus den entsprechenden Anhydriden hergestellt.

Polyisobutylbernsteinsäureanhydride wiederum werden im üblicherweise aus Polyisobutenen und Maleinsäureanhydrid hergestellt. Zur Ausbeutesteigerung wird diese Reaktion häufig in Gegenwart von Chlor vorgenommen, was zur Folge hat, daß chlorhaltige Nebenprodukte entstehen. Solche Produkte sind heute unerwünscht, da sie aufgrund ihrer Nebenbestandteile korrosionsfördernd sein können.

Eine rein thermische Umsetzung von Polyisobuten und Maleinsäureanhydrid kann zu chlorfreien Produkten führen. Jedoch sind dazu nach der Lehre der EP-A 457 599 relativ hohe Temperaturen von über 200°C in Verbindung mit Überdruck nötig, um wirtschaftliche Reaktionszeiten zu erzielen. Unter diesen Reaktionsbedingungen kommt es zu Teerbildung, die die Qualität des Produktes erniedrigt. Weiterhin tritt bei diesen hohen Temperaturen leicht eine teilweise Bismaleinierung des Polyisobutens auf. Diese Folgereaktion des Polyisobutenbernsteinsäureanhydrids ist ebenfalls unerwünscht im Hinblick auf eine Verwendung der Produkte oder daraus hergestellter Imide in Kraftstoffen, da solche Produkte mit einem Bismaleinierungsgrad von über 10 % die Ablagerungsraten auf Ventilen erhöhen können.

Der Erfindung lag ein verbessertes Verfahren zur Herstellung von Polyisobutylbernsteinsäureanhydriden als Aufgabe zugrunde, das es erlaubt, unter verfahrenstechnisch einfach zu realisierenden milden Reaktionsbedingungen zu Produkten zu gelangen, die möglichst teerfrei anfallen und weiterhin minimale Bismaleinierungsgrade aufweisen.

Demgemäß wurde ein verbessertes Verfahren zur Herstellung von Polyisobutylbernsteinsäureanhydriden aus Polyisobuten und Maleinsäureanhydrid gefunden, das dadurch gekennzeichnet ist, daß man Polyisobuten mit einem mittleren Molekulargewicht M_{N} von 600 - 5000 und einem Gehalt an endständigen Doppelbindungen von mindestens 50 % mit Maleinsäureanhydrid in einer Reinheit von mindestens 99 % im Verhältnis von 1,1 : 1 bis 6 : 1 mol Maleinsäureanhydrid zu Polyisobuten bei 140 - 200°C umsetzt. Weiterhin wurde ein Verfahren zur Herstellung von Polyisobutylbernsteinsäureamiden und -imiden aus den Anhydriden gemäß dem beschriebenen Verfahren gefunden, sowie die Verwendung der genannten Amide und Imide als Zusatzstoffe für Kraftstoffe und Schmieröle. Schließlich wurden Kraftstoffe und Schmieröle gefunden, die die genannten Verfahrensprodukte enthalten.

Die erfindungsgemäß zu verwendenden Polyisobutene sind bekannt (z.B. aus der EP-A 145 235) und weisen bei einem Molekulargewicht M_{N} von 600 - 5000, vorzugsweise 1000 - 2500 einen Gehalt an endständigen Doppelbindungen von mindestens 50 %, vorzugsweise 70 - 90 % auf. Diese endständige Doppelbindungen sind im Vergleich zu innenständigen Doppelbindungen bei Umsetzungen mit Maleinsäureanhydrid besonders reaktiv.

Diese Polyisobutene werden mit Maleinsäureanhydrid umgesetzt. Um teerfreie Produkte zu erlangen, wird Maleinsäureanhydrid mit hoher Reinheit, vorzugsweise mit einer Reinheit von mindestens 99 %, besonders von mindestens 99,5 % eingesetzt.

Die Ausgangsstoffe werden im molaren Verhältnis von 1,1 : 1 bis 6 : 1, vorzugsweise 1,2 : 1 bis 3 : 1 von Maleinsäureanhydrid zu Polyisobuten umgesetzt.

Die Reaktion wird bei 140 - 200°C, vorzugsweise bei 150 - 190°C vorgenommen. Im allgemeinen herrscht bei Reaktion Normaldruck, es kann jedoch auch unter dem Eigendruck des Reaktionsgemisches oder bei erhöhtem Druck gearbeitet werden.

Die Reaktion kann in Substanz, aber auch in Gegenwart eines Lösungsmittels vorgenommen werden. Es sind hier besonders hochsiedende Aromaten wie Toluol, Xylol und Naphtha, weiterhin Ether wie Dimethyldiglykol und Diethyldiglykol zu nennen. Üblicherweise beträgt die Menge des verwendeten Lösungsmittels 5 bis 30 Gew.-% bezogen auf den Reaktionsansatz. Ein Teil des Lösungsmittels kann beim Aufheizen des Reaktionsansatzes auf die Reaktionstemperatur abdestilliert werden.

Die Ausgangsstoffe können vor der Reaktion vermischt und bei der Reaktionstemperatur umgesetzt werden. In einer besonders bevorzugten Ausführungsform wird nur ein Teil des Maleinsäureanhydrids vorgelegt und der verbleibende Teil wird der Reaktionsmischung bei Reaktionstemperatur so zugesetzt, daß immer eine homogene Phase im Reaktionsgefäß vorliegt, durch die eine Umsetzung ohne Bildung wesentlicher Mengen an Nebenprodukten erleichtert wird.

Die Reaktion wird vorteilhaft in inerter Atmosphäre, z.B. unter Stickstoff, durchgeführt.

Je nach der gewählten Reaktionstemperatur und dem Überschuß an Maleinsäureanhydrid ist die Reaktion in der Regel nach 2 bis 16 Stunden beendet. In an sich bekannter Weise wird daraufhin auf das Verfahrensprodukt aufgearbeitet. Im allgemeinen werden dazu alle flüchtigen Bestandteile abdestilliert und der Destillationsrückstand wird isoliert.

Die Farbe des nach dem erfindungsgemäßen Verfahren hergestellten Polyisobutylbernsteinsäureanhydrids ist gelblich bis bernsteinfarben. Auch der Reaktor zeigt keinen Teerbelag nach der Beendigung der Reaktion. Der Bismaleinierungsgrad des Reaktionsproduktes ist so gering, daß das Verhältnis von Bernsteinsäureanhydridgruppen zu Polyisobuteneinheiten unter 1,1:1 liegt (d.h. daß der Bismaleinierungsgrad unter 10 % liegt), vorzugsweise sogar unter 1,05:1.

Der Polyisobutenumsatz liegt in der Regel unter dem Umsatz, der bei vollständiger Reaktion aller endständigen Doppelbindungen zu erwarten wäre.

Die nach dem erfindungsgemäßen Verfahren hergestellten Polyisobutylbernsteinsäureanhydride dienen als Zwischenprodukte für die Herstellung von Polyisobutylbernsteinsäureamiden und -imiden.

Diese Produkte werden in an sich bekannter Weise durch Umsetzung von Polyisobutylbernsteinsäureanhydriden mit einem primären oder sekundären Amin unter Abspaltung von Wasser umgesetzt.

Bei diesen Aminen kann es sich prinzipiell um alle zur Amid- und Imidbildung geeigneten Verbindungen handeln wie Ammoniak, mono- und dialiphatische Amine, cycloaliphatische und aromatische Amine. Im Hinblick auf die Verwendung der Verfahrensprodukte als Zusatz zu Kraftstoffen und Schmierstoffen sind jedoch Polyamine bevorzugt. Die Polyamine tragen vorzugsweise 2 bis 10 Stickstoffatome. Besonders bevorzugt sind solche Polyamine, die Alkylengruppen tragen wie Ethylen, 1,2-Propylen, 2,2-Dimethylpropylen, z.B. Ethylendiamin, Diethylentriamin, Triethylentetramin, Tetraethylenpentamin, Dipropylentriamin und Tripropylentetramin.

Die erfindungsgemäß erhältlichen Polyisobutylbernsteinsäureamide und -imide finden als Zusatz für Otto-, Wankel- und Diesel-Kraftstoffe Verwendung, insbesondere für Kraftstoffe in Ottomotoren, vorzugsweise in Mengen von 20 bis 300 ppm reiner Wirksubstanz bezogen auf den Kraftstoff. Sie können auch in Kombination mit anderen Dispergatoren wie Polyisobutylaminen verwendet werden.

Weiterhin eignen sie sich als Zusätze für Schmierstoffe, insbesondere mineralische, teilsynthetische und vollsynthetische Motoröle, wie Motoröle der gehobenen Leistungsklasse APJ SG/CE, vorzugsweise in Mengen von 1 bis 10 Gew.-% bezogen auf das Öl.

### Beispiele

### Herstellung von Polyisobutylbernsteinsäureanhydriden

### Beispiel 1

3 kg (2,96 Mol) Polyisobuten mit einem Gehalt an endständigen Doppelbindungen von 85 % und einem mittleren Molekulargewicht M_{N} von 1013 wurden mit 150 g Maleinsäureanhydrid und 600 g Naphtha (Siedelage 150 - 180°C) versetzt und auf 180°C erhitzt. Innerhalb von 2 Stunden wurden weitere 300 g Maleinsäureanhydrid (Reinheit von 99,6 %, molares Verhältnis Maleinsäureanhydrid zu Polyisobuten 1,55 : 1) zugesetzt. Das Reaktionsgemisch wurde 6 Stunden bei dieser Temperatur gerührt. Lösungsmittel und nicht umgesetztes Maleinsäureanhydrid wurden abdestilliert und der Destillationsrückstand wurde isoliert.

Das Verfahrensprodukt fiel als klare bernsteinfarbene Flüssigkeit mit einer Hazen-Farbzahl von 370 (Bestimmung nach DIN ISO 6271) an. Der Bismaleinierungsanteil betrug 2 % (Berechnung aus Umsatz des Polyisobutens, Molekulargewicht und Verseifungszahl des Produktes). Der Polyisobutenumsatz betrug 74 %.

### Beispiel 2

Die Umsetzung wurde in Analogie zu Beispiel 1 vorgenommen, jedoch wurden beim Aufheizen des Reaktionsgemisches bereits 50 g Lösungsmittel abdestilliert. Nach destillativer Aufarbeitung wurde ein gelbliches Produkt mit einer Hazen-Farbzahl von 250 und einem Bismaleinierungsgrad von 1 % isoliert. Der Polyisobutenumsatz betrug 80 %.

### Beispiel 3

Das Beispiel wurde in Analogie zu Beispiel 2 durchgeführt, jedoch unter Verwendung eines Lösungsmittelgemisches aus 120 g Naphtha und 480 g Toluol. Nach destillativer Aufarbeitung wurde ein gelbliches Produkt isoliert, das eine Hazen-Farbzahl von 240 und einen Bismaleinierungsgrad von 0 % aufwies. Der Polyisobutenumsatz betrug 78 %.

### Beispiel 4

Das Beispiel wurde in Anlehnung an Beispiel 1 durchgeführt. Die Reaktion wurde jedoch ohne Lösungsmittel bei 155°C vorgenommen. Die Gesamtmenge an Maleinsäureanhydrid wurde über 2 Stunden bei der Reaktionstemperatur zugegeben. Das Reaktionsgemisch wurde 14 Stunden bei dieser Temperatur gehalten.

Nach destillativer Aufarbeitung erhielt man ein gelbliches Produkt mit einer Hazen-Farbzahl von 210 und einem Bismaleinierungsgrad von 1 %. Der Polyisobutenumsatz betrug 78 %.

### Beispiel 5

Das Beispiel wurde in Anlehnung an Beispiel 1 durchgeführt, jedoch bei einer Reaktionstemperatur von 195°C und einem Maleinsäureanhydrid-Zulauf von 160 g in einer halben Stunde. Die Reaktionszeit betrug 3 Stunden. Das isolierte Produkt wies eine Hazen-Farbzahl von 240 und einen Bismaleinierungsanteil von 3 % auf. Der Polyisobutenumsatz betrug 83 %.

### Vergleichsbeispiel 1

Das Beispiel wurde in Analogie zu Beispiel 5 durchgeführt, jedoch bei einer Reaktionstemperatur von 225°C.

Nach Aufarbeitung wurde ein schwach-trübes, dunkelbraunes Produkt isoliert, dessen Farbzahl nicht bestimmt werden konnte. Der Bismaleinierungsanteil betrug 31 %. Am Rührer und an den Reaktorwänden waren kleine Mengen schwarz-brauner, teerartiger Rückstände erkennbar, die acetonlöslich waren. Der Polyisobutenumsatz betrug 90 %.

### Herstellung von Polyisobutylbernsteinsäureimiden

### Beispiel 6

703 g Polyisobutylbernsteinsäureanhydrid nach Beispiel 2 wurden zu 37,5 g Triethylentetramin in 270 g Naphtha bei 180°C gegeben und 30 min. unter Abdestillieren von Wasser erhitzt. Das bernsteinfarbene Produkt wies eine Säurezahl von unter 1 mg KOH pro g Substanz auf.

### Vergleichsbeispiel 2

620 g Polyisobutylbernsteinsäureanhydrid nach Vergleichsbeispiel 1 wurden bei 180°C zu 47,7 g Triethylentetramin in 340 g Naphtha gegeben und unter Abdestillieren von Wasser 30 min. erhitzt. Man erhielt ein trübes, schwarz-braunes Imid mit einer Säurezahl von unter 1 mg KOH pro g Substanz.

### Motorentest

Der Motortest wurde in einem 1,05 l Polomotor durchgeführt. Eingesetzter Kraftstoff: bleifreier Kraftstoff nach DIN 51607. Es wurde auf Reinhaltung der Einlaßventile gemäß folgendem Programm über 40 Stunden (71 Zyklen) geprüft:

| | | |
|---|---|---|
| 4 Minuten: | 1000 Umdrehungen pro Minute, | 3,3 KW Last |
| 10 Minuten: | 3000 Umdrehungen pro Minute, | 22,1 KW Last |
| 6 Minuten: | 5300 Umdrehungen pro Minute, | 29,5 KW Last |
| 11 Minuten: | 4000 Umdrehungen pro Minute, | 23,6 KW Last |
| 3 Minuten: | 2500 Umdrehungen pro Minute, | 5,5 KW Last |

Nach dem Prüflauf wurden die Einlaßventile ausgebaut, mit Cyclohexan gewaschen und die Masse der nicht abgelösten Ablagerungen bestimmt.

Zur Prüfung der Brennraumablagerungen wurden die Rückstände in den Brennräumen der Zylinderköpfe und von den Kolbenböden gesammelt.

**Tabelle**

| Additiv | Dosierung in ppm | Einlaßventilablagerung in mg (Durchschnittswert pro Ventil) | Rückstände in g |
|---|---|---|---|
| Polyisobutylbernsteinsäureimid nach Beispiel 6 | 600 | 0 | 3,58 |
| Polyisobutylbernsteinsäureimid nach Beispiel 6 | 400 | 0 | 3,62 |
| Polyisobutylbernsteinsäureimid nach Vergleichsbeispiel 2 | 600 | 61 | 4,79 |
| ohne Zusatz | - | 265 | 3,76 |

Die erfindungsgemäße Verbindung weist eine ausgezeichnete Wirkung als Detergenz auf und verhindert jegliche Ablagerung an den Einlaßventilen. Die Beispiele zeigen klar, daß durch das erfindungsgemäße Imid mit geringem Bismaleinierungsanteil keine zusätzlichen Rückstände im Brennraum wie beim Produkt aus Vergleichsbeispiel 2 gebildet werden.

### Tüpfeltest

Es wurden 3 gew.-%ige Mischungen der Polyisobutylbernsteinsäureimide mit Rußdispersionen in einem Mineralöl hergestellt. Die Proben wurden bei Raumtemperatur RT oder bei 250°C 10 Minuten getempert.

Die so erhaltene Dispersion wurde auf einem Filterpapier wie ein Chromatogramm entwickelt. Die Werteskala reicht von 0 bis 1 000, wobei 1 000 100 % Dispergierwirkung entspricht.

| | RT | 250°C |
|---|---|---|
| Polyisobutylbernsteinsäureimid nach Beispiel 6 | 680 | 650 |
| Imid nach Vergleichsbeispiel 2 | 600 | 580 |

Der Versuch zeigt, daß die dispergierenden Eigenschaften der erfindungsgemäß hergestellten Verbindung denen der Vergleichsverbindung überlegen sind. (Testbeschreibung: "Les Huiles pour Moteurs et le Graissage des Moteurs", A. Schilling Vol. 1, Seite 89 f., 1962).

## Patentansprüche

1. Verfahren zur Herstellung von Polyisobutylbernsteinsäureanhydriden aus Polyisobuten und Maleinsäureanhydrid, dadurch gekennzeichnet, daß man Polyisobuten mit einem mittleren Molekulargewicht M_{N} von 600 - 5000 und einem Gehalt an endständigen Doppelbindungen von mindestens 50 % mit Maleinsäureanhydrid in einer Reinheit von mindestens 99 % im Verhältnis von 1,1 : 1 bis 6 : 1 mol Maleinsäureanhydrid zu Polyisobuten bei 140 - 200°C umsetzt.

2. Verfahren nach Anspruch 1, dadurch gekennzeichnet, daß das mittlere Molekulargewicht M_{N} des Polyisobutens 1000 bis 2500 beträgt.

3. Verfahren nach Anspruch 1 oder 2, dadurch gekennzeichnet, daß der Gehalt an endständigen Doppelbindungen im Polyisobuten 70 - 90 % beträgt.

4. Verfahren nach den Ansprüchen 1 bis 3, dadurch gekennzeichnet, daß man die Reaktion in homogener Phase vornimmt.

5. Verfahren zur Herstellung von Polyisobutylbernsteinsäureamiden und -imiden, dadurch gekennzeichnet, daß man die nach den Verfahren gemäß den Ansprüchen 1 - 4 erhältlichen Polyisobutylbernsteinsäureanhydride mit einem aliphatischen Polyamin mit 2 bis 10 Stickstoffatomen umsetzt.

6. Additive für Kraftstoffe und Schmierstoffe, erhältlich nach dem Verfahren gemäß Anspruch 5.

7. Verwendung von Polyisobutylbernsteinsäureamiden und -imiden, erhältlich nach dem Verfahren gemäß Anspruch 5, als Additiv in Kraftstoffen und Schmierstoffen.

8. Kraftstoffe, enthaltend geringe Mengen an Polyisobutylbernsteinsäureamiden oder -imiden, erhältlich nach dem Verfahren gemäß Anspruch 5.

9. Schmierstoffe, enthaltend Polyisobutylbernsteinsäureamide oder -imide, erhältlich nach dem Verfahren gemäß Anspruch 5.
